# EUROPEAN PATENT APPLICATION

(11) **EP 4 686 456 A1**
(43) Date of publication of application: **04.02.2026**
(21) Application number: 24217761.6
(22) Date of filing: 05.12.2024
(51) Int. Cl.: A61B 50/10

(54) **CABINET FOR STORING SURGICAL INSTRUMENTS AND METHOD FOR OPERATING A CABINET FOR STORING SURGICAL INSTRUMENTS**

(30) Priority: 29.07.2024 DE 102024121513
(71) Applicant: OLYMPUS WINTER & IBE GMBH, 22045 Hamburg (DE)
(72) Inventor: HOPF, Oliver, 25421 Pinneberg (DE)
(74) Representative: Seemann & Partner Patentanwälte mbB

(57) **Abstract**

The invention relates to cabinet 1 for storing surgical instruments 20 as well as a method for operating the cabinet 1. The cabinet 1 comprises a ventilation unit 8 and a casing 2. The casing 2 encloses an interior space 4 with at least one mounting device 7. The mounting device 7 is configured to hold a surgical instrument 20. The ventilation unit 8 is configured to supply drying gas 10 to the interior space 4. The cabinet 1 further comprised a cooling unit 9 connected to the ventilation unit 8. The cooling unit 9 is configured to cool down the drying gas 10 before it enters the interior space 4 to a temperature below 15°C.

## Description

The invention relates to a cabinet for storing surgical instruments. The invention further relates to a method for operating a cabinet for storing surgical instruments.

Surgical instruments, for example endoscopes, have to be cleaned and disinfected after use. After cleaning, it is necessary to dry the surgical instruments to remove any traces of the cleaning fluid, before they can be used again. For this drying process, drying cabinets are often used, in which the surgical instruments are arranged after cleaning.

However, sometimes the surgical instruments will stay in the drying cabinet for several days or weeks before they are required again. If the surgical instruments remain in the drying cabinet for such a long period of time, there is a risk that they are contaminated again through germs transmitted through ambient air or by touch. Such germs, which might get into contact with a surgical endoscope after disinfection, may include for example Escherichia coli, Pseudomonas aeruginosa, Staphylococcus aureus, streptococci, Candida albicans or Aspergillus fumigatus. All these types of germs are potentially harmful during an operation and may contaminate the surgical instruments while they are stored inside the drying cabinet.

It is an object of the present invention to better protect surgical instruments from recontamination by germs after cleaning and disinfection during the drying or storage phase.

This object is solved by a cabinet for storing surgical instruments, in particular endoscopes, wherein the cabinet comprises a ventilation unit and a casing, wherein the casing encloses an interior space with at least one mounting device, wherein the mounting device is configured to hold a surgical instrument, wherein the ventilation unit is configured to supply drying gas to the interior space, characterized in that the cabinet further comprises a cooling unit and a drying unit connected to the ventilation unit, wherein the drying unit is configured to reduce the humidity of the drying gas, wherein the cooling unit is configured to cool down the drying gas before it enters the interior space to a temperature below 15°C.

Advantageously, by cooling down the drying gas before it enters the interior space, the probability of recontamination with germs is sharply reduced. The reason for this is that the types of germs, which are typically responsible for the recontamination, all thrive poorly at low temperatures. Thus, by cooling down the drying gas, the environmental conditions for the germs are strongly impaired.

The drying unit improves water absorption and thus reduces the humidity of the drying gas in order to improve the drying capacity of the cabinet. The drying unit may be a passive drying unit. For example, the drying unit may comprise a silicate mineral in order to passively dry the drying gas. The drying unit may be an active drying unit. For example, the drying unit may comprise an air drying configured to actively dry the drying gas. In particular, the cooling unit and the drying unit may be formed as a single cooling and drying unit.

The drying gas may be air, a noble gas or a mixture of different gases. The cabinet may comprise a control system for controlling the ventilation unit and the cooling unit and the drying unit.

Preferably, the cooling unit is configured to cool down the drying gas before it enters the interior space to a temperature below 10°C, in particular to a temperature of 6°C to 8°C. A temperature of about 7°C is particularly advantageous, as it greatly inhibits the recontamination with germs and offers good storage conditions for the surgical instruments.

Preferably, the interior space is thermally insulated and airtight against the exterior of the cabinet. The thermal insulation and the airtight design make the operation of the cooling unit much more efficient, so that it takes less energy to cool down the drying gas and keep it at low temperatures. In addition, the recontamination of surgical instruments by germs from the exterior is prevented by the airtight design.

According to an embodiment, the cabinet comprises at least one door, wherein the door is sealed against the casing with an airtight sealing in a closed state. The airtight sealing prevents ambient air from entering the interior space through small gaps between the door and the casing. The door may have two door wings.

Preferably, the cabinet comprises a closed-off circulation system for the drying gas, wherein the closed off circulation system comprises the interior space, the ventilation unit and the cooling unit. In other words, the cabinet provides a closed loop for the drying gas inside the cabinet. In particular, there is no air intake for ambient air and no intake for gas from outside the cabinet. Instead, the drying gas is continuously circulated through the interior space, the ventilation unit and the cooling unit in order to keep the temperature of the drying gas inside the interior space cool. This not only prevents the recontamination via germs from the outside but also saves energy during the operation of the cabinet.

Preferably, the ventilation unit is configured to circulate the drying gas between the interior space and the cooling unit and the drying unit. The ventilation unit may comprise one or more ventilation devices to circulate the drying gas. The ventilation devices may comprise at least one ventilator or fan.

According to an embodiment, the drying unit and/or the ventilation unit and/or the cooling unit comprise a filter, wherein the filter is configured to continuously filter the drying gas. By continuously filtering the drying gas, any remaining germs and other contaminations are removed from the drying gas during the operation of the cabinet. Thus, the filter prevents recontamination of the surgical instruments. In particular, the filter is a sterile filter. In particular, the filter is configured to filter the drying gas coming from the interior space, before the filtered gas is guided back towards the interior space or towards the cooling unit.

Preferably, the ventilation unit is configured to supply the drying gas as a drying gas stream to at least one interior channel of a surgical instrument held in the mounting device. By supplying the drying gas stream to the interior channels of the surgical instrument, these interior channels are also provided with cooled-down drying gas. In this way, the cabinet prevents recontamination of the outer surface and the interior channels of the surgical instrument.

According to an embodiment, the cooling unit is configured to cool down the drying gas during a storage phase, which happens after a drying phase. The drying phase is a phase during the operation of the cabinet immediately after the surgical instrument is placed in the interior space. Typically, the surgical instrument is still wet from the cleaning process and needs to be dried. Thus, during the drying phase, the drying gas is utilized to remove any remaining cleaning fluid. During the storage phase, which happens after the drying phase, the drying of the surgical instrument has already finished and the surgical instrument is stored inside the cabinet until its next use. It is advantageous to cool down the drying gas doing the storage phase as a recontamination typically happens during this phase, as the storage phase may last a lot longer than the drying phase. In particular, the cooling unit and/or the control system is configured to operate in a way, that a temperature of the drying gas is higher during the drying phase than during the storage phase. This is advantageous, because during the drying phase a higher temperature accelerates the removal of the remaining cleaning fluid. In particular, an air pressure of the drying gas is higher during the drying phase than during the storage phase.

According to an embodiment, the cabinet comprises multiple mounting devices. In particular, the ventilation unit is configured to provide each mounting unit separately with the drying gas. The cabinet may comprise a separate vent or nozzle for each mounting device. The vents or nozzles are in particular arranged in proximity to the associated mounting devices. In particular, the vents or nozzles are configured to create a separate drying gas stream for each surgical instrument arranged in the mounting devices. In this way, the surgical instruments may be supplied with drying gas streams with different properties. For example, a surgical instrument that is still wet may be supplied with a stronger drying gas stream, whereas a surgical instrument in the storage phase may be supplied with a weaker drying gas stream.

The object is further solved by a method for operating a cabinet for storing surgical instruments, in particular endoscopes, wherein the cabinet comprises a ventilation unit and a casing, wherein the casing encloses an interior space with at least one mounting device, wherein the mounting device holds a surgical instrument, wherein the ventilation unit supplies drying gas to the interior space, characterized in that the cabinet further comprises a cooling unit connected to the ventilation unit, wherein the cooling unit cools down the drying gas before it enters the interior space to a temperature below 15°C.

The method embodies the same advantages and characteristics as the cabinet described above.

Preferably, the cooling unit cools down the drying gas before it enters the interior space to a temperature below 10°C, in particular to a temperature of 6°C to 8°C.

Preferably, the cabinet circulates the drying gas between the interior space and the cooling unit.

According to an embodiment, the drying unit and/or the ventilation unit and/or the cooling unit comprises a filter, wherein the filter continuously filters the drying gas.

Preferably, the ventilation unit supplies the drying gas as a drying gas stream to at least one interior channel of the surgical instrument held in the mounting device.

Preferably, the cooling unit cools down the drying gas during a storage phase, which happens after a drying phase.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
Fig. 1 shows a schematic simplified perspective representation of a cabinet for storing surgical instruments, and
Fig. 2 shows a schematic simplified close-up of the cabinet and several surgical instruments stored within.

In the drawings, the same or similar types of elements or respectively corresponding parts are provided with the same reference numbers in order to prevent the item from needing to be reintroduced.

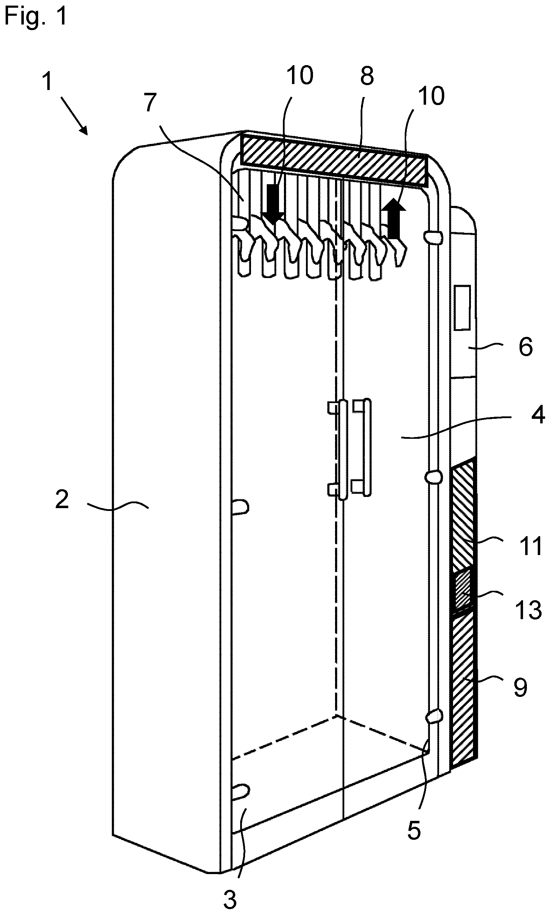

Fig. 1 shows a schematic, simplified perspective drawing of a cabinet 1 for drying and storing surgical instruments. The cabinet 1 comprises a casing 2 and a door 3 with wings. The casing 2 and the door 3 enclose an interior space 4. The interior space 4 is thermally insulated and airtight against the outside of cabinet 1. This is partly achieved by an airtight sealing 5 between the door 3 and the casing 2.

Inside the interior space 4, there are a number of mounting devices 7, which are configured to hold a surgical instrument each, for example an endoscope. The cabinet further comprises a ventilation unit 8, which is configured to supply drying gas 10 to the interior space 4 and circulate the drying gas 10 between interior space 4 and a cooling unit 9. The cooling unit 9 is configured to cool down the drying gas 10 to a temperature of about 7°C. Such a temperature is advantageous, because it prevents a recontamination of the surgical instrument stored inside the interior space 4. The drying gas 10 is circulated in a closed loop inside the cabinet 1 between the cooling unit 9, the ventilation unit 8 and interior space 4. A control system 6, which may comprise a screen, control elements and a computer or a similar device, controls the operation of the cabinet, for example by controlling the ventilation unit 8 and the cooling unit 9. The cabinet 1 further comprises a drying unit 11. The drying unit 11 may be a separate unit or a single cooling and drying unit together with the cooling unit 9. The drying unit 11 dries the drying gas 10 in order to improve the drying capacity of the cabinet 1. The drying unit 11 and/or the ventilation unit 8 and/or the cooling unit 9 may comprise one or more filter 13 in order to filter contaminations from the drying gas 10.

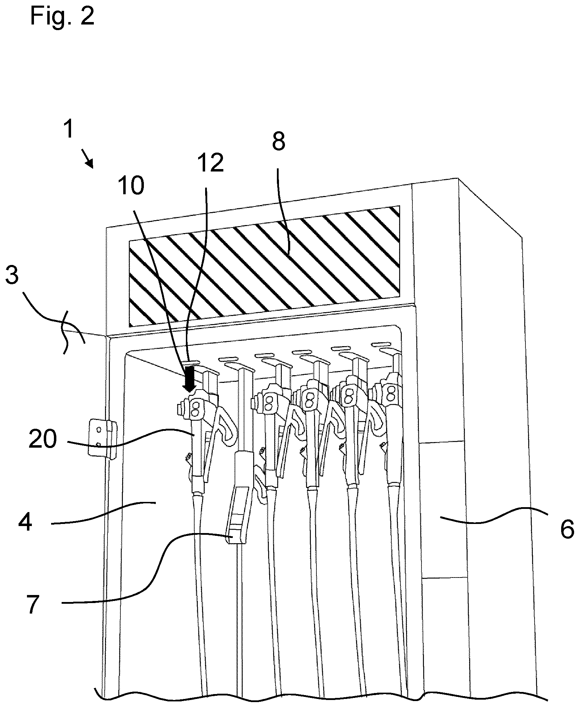

Fig. 2 shows a close-up perspective of the cabinet 1. In Fig. 2, several of the mounting devices 7 each hold a surgical instrument 20, in this case an endoscope. For simplicity sake, only one of the surgical instruments 20 and only one of the mounting devices 7 are provided with reference signs in Figs. 1 and 2. As can be seen in Fig.2, the cabinet 1 may comprise several vents 12 or nozzles. The vents 12 guide the drying gas 10 into the interior space 4 and may also guide the drying gas 10 back to the ventilation unit 8.

In order to protect the in interior channels of the surgical instruments 20 from recontamination, the drying gas 10 may also be guided as a drying gas steam through in the interior channels of the surgical instruments 20. In order to guide the drying gas stream, the cabinet 1 may comprise a connection device, for example a connection tube, which is not shown in Fig. 2.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 1: cabinet
- 2: casing
- 3: door
- 4: interior space
- 5: sealing
- 6: control system
- 7: mounting device
- 8: ventilation unit
- 9: cooling unit
- 10: drying gas
- 11: drying unit
- 12: vents
- 13: filter
- 20: surgical instrument

## Claims

1. Cabinet (1) for storing surgical instruments (20), in particular endoscopes, wherein the cabinet (1) comprises a ventilation unit (8) and a casing (2), wherein the casing (2) encloses an interior space (4) with at least one mounting device (7), wherein the mounting device (7) is configured to hold a surgical instrument (20), wherein the ventilation unit (8) is configured to supply drying gas (10) to the interior space (4), **characterized in that** the cabinet (1) further comprises a cooling unit (9) and a drying unit (11) connected to the ventilation unit (8), wherein the drying unit (11) is configured to reduce the humidity of the drying gas, wherein the cooling unit (9) is configured to cool down the drying gas (10) before it enters the interior space (4) to a temperature below 15°C.

2. Cabinet (1) according to claim 1, wherein the cooling unit (9) is configured to cool down the drying gas (10) before it enters the interior space (4) to a temperature below 10°C, in particular to a temperature of 6°C to 8°C.

3. Cabinet (1) according to claim 1 or 2, wherein the interior space (4) is thermally insulated and airtight against an exterior of the cabinet (1).

4. Cabinet (1) according to any of claims 1 to 3, comprising at least one door (3), wherein the door (3) is sealed against the casing (2) with an airtight sealing (5) in a closed state.

5. Cabinet (1) according to any of claims 1 to 4, comprising a closed off circulation system for the drying gas (10), wherein the closed off circulation system comprises the interior space (4), the ventilation unit (8) and the cooling unit (9).

6. Cabinet (1) according to any of claims 1 to 5, wherein the ventilation unit (8) is configured to circulate the drying gas (10) between the interior space (4) and the cooling unit (9) and the drying unit (11).

7. Cabinet (1) according to one to any of claims 1 to 6, wherein the drying unit (11) and/or ventilation unit (8) and/or the cooling unit (9) comprises a filter (13), wherein the filter (13) is configured to continuously filter the drying gas (10).

8. Cabinet (1) according to any of claims 1 to 7, wherein the ventilation unit (8) is configured to supply the drying gas (10) as a drying gas stream to at least one interior channel of a surgical instrument (20) held in the mounting device (7).

9. Cabinet (1) according to any of the claims 1 to 8, wherein the cooling unit (9) is configured to cool down the drying gas during a storage phase, which happens after a drying phase.

10. Method for operating a cabinet (1) for storing surgical instruments (20), in particular endoscopes, wherein the cabinet (1) comprises a ventilation unit (8) and a casing (2), wherein the casing (2) encloses an interior space (4) with at least one mounting device (7), wherein the mounting device (7) holds a surgical instrument (20), wherein the ventilation unit (8) supplies drying gas (10) to the interior space (4), **characterized in that** the cabinet (1) further comprises a cooling unit (9) connected to the ventilation unit (8), wherein the cooling unit (9) cools down the drying gas (10) before it enters the interior space (4) to a temperature below 15°C.

11. Method according to claim 10, wherein the cooling unit (9) cools down the drying gas (10) before it enters the interior space (4) to a temperature below 10°C, in particular to a temperature of 6°C to 8°C.

12. Method according to claim 10 or 11, wherein the cabinet (1) circulates the drying gas (10) between the interior space (4) and the cooling unit (9).

13. Method according to any of the claims 10 to 12, wherein the drying unit (11) and/or ventilation unit (8) and/or the cooling unit (9) comprises a filter (13), wherein the filter (13) continuously filters the drying gas (10).

14. Method according to any of the claims 10 to 13, wherein the ventilation unit (8) supplies the drying gas (10) as a drying gas stream to at least one interior channel of the surgical instrument (20) held in the mounting device (7).

15. Method according to any of the claims 10 to 14, wherein the cooling unit (9) cools down the drying gas (10) during a storage phase, which happens after a drying phase.
